# EUROPEAN PATENT APPLICATION

(11) **EP 3 621 083 A1**
(43) Date of publication of application: **11.03.2020**
(21) Application number: 18193490.2
(22) Date of filing: 10.09.2018
(51) Int. Cl.: G16H 20/30, A61B 5/11

(54) **REHABILITATION DEVICE AND METHOD**

(71) Applicant: Koninklijke Philips N.V., 5656 AE Eindhoven (NL)
(72) Inventor: JIN, Sheng, 5656 AE Eindhoven (NL); WANG, Jin, 5656 AE Eindhoven (NL)
(74) Representative: de Haan, Poul Erik

(57) **Abstract**

A rehabilitation device for a rehabilitation exercise. The device comprises at least one marker configured to be attached to at least one body part of a subject. The device comprises an image capture unit configured to capture a first image of the at least one marker at a first position and a second image of the at least one marker at a second position. The device comprises a processor configured to receive the first image and the second image from the image capture unit, identify a first region of interest (ROI-1) of the at least one marker in the first image, identify a second region of interest (ROI-2) of the the at least one marker in the second image, compare the first ROI (ROI-1) with the second ROI (ROI-2), and output data based on at least the comparing results. A computer-implemented method is proposed for rehabiliatation exercise. A computer program product is proposed for operating a rehabilitation device

## Description

### FIELD OF THE INVENTION

The invention relates to a rehabilitation device for a rehabilitation exercise. The invention further relates to a method of operating a rehabilitation device, and to a computer program product for operating such a rehabilitation device.

### BACKGROUND OF THE INVENTION

A camera based stroke rehabilitation system is employed to give guidance/feedback when the patients are doing rehabilitation exercise by detecting key joint's moving trajectory using algorithms to analyze images captured by the camera.

The camera continuously captures images of the markers which are placed, for example, at the hand, the elbow or the shoulder. These markers are easily distinguished in the captured images because the reflective materials make these markers brighter than other parts of the body and background environment.

Hand function and fine motor skills are often impaired after having a stroke. A hand rehabilitation exercise called plamer and dorsal flexion is beneficial in improving strength and dexterity. A flexion/rotation angle between the palm and the forearm which defines a range of movement of the plamer and dorsal flexion exercise is determined by analyzing captured images of the marker attached to the hand.

Conventionally, an area-angle mapping table is applied to determine the angle by calculating the size of a corresponding area of the markers in the captured images. However, the area of the hand marker in the captured images may be impacted by several factors illustrated as below. Under some circumstances, hand marker and elbow marker may be overlapped or may interfere each other. Thus, the area of the targeted hand marker includes unexpected redundant information. Under some circumstances, unexpected hole areas may be distributed in the hand maker area in the captured images due to the unfavorable surface conditions of the hand marker, light may not be properly or sufficently reflected onto the camera such that the camera may miss some information of the hand marker.

### SUMMARY OF THE INVENTION

It is an object of the invention to provide an improved rehabilitation device and method to resolve the above mentioned problems during a rehabilitation exercise so as to improve the reliability and accurancy of the rehabilitatioan device and speed up the rehabilitation process.

For this purpose, according to a first aspect of the invention, a rehabilitation device for a rehabilitation exercise is provided, the device comprising:
at least one marker configured to be attached to at least one body part of a subject;
an image capture unit configured to capture a first image of the at least one marker at a first position and a second image of the at least one marker at a second position; and
a processor configured to:
   receive the first image and the second image from the image capture unit;
   identify a first region of interest (ROI) of the at least one marker in the first image;
   identify a second region of interest (ROI) of the at leat one marker in the second image;
   compare the first ROI with the second ROI; and
   output data based on at least the comparing results.

For this purpose, according to a second aspect of the invention, a computer-implemented method for rehabiliatation exercise, comprising:
receiving a first image and a second image;
identifying a first region of interest (ROI) of at least one marker in the first image;
identifying a second region of interest (ROI) of the at leat one marker in the second image;
comparing the first ROI with the second ROI; and
outputting data based on at least the comparing results.

For this purpose, according to a third aspect of the invention, a computer program product for operating a rehabilitation device, which program is operative to cause a processor to perform the method as described above.

The at least one marker may be attached to the hand, the elbow or the shoulder of the subject. The marker may also be attached to the leg, the knee joint, the hip or other body part of the subjuect where exercise trajectory is needed to be determined and tracked.

The first ROI may be a point, a line or an area determined by at least part of a boundary of the hand marker in the first image in 2D, 3D or 4D space. The second ROI may be a point, a line or an area determined by at least part of a boundary of the hand marker in the second image in 2D, 3D or 4D space. The first ROI and the second ROI are identified based on the boundary of the hand marker instead of being calculated based on the area size of the hand maker. In this way, the data (e.g., a flextion/rotation angle) calcualted based on the comparison results obtained by comparing the first ROI with the second ROI is more accurate and reliable as compared to the calculation algorithms based on the area-angle mapping table in the prior art.

In various embodiments, the step of identifying the first ROI comprises calculating a first point indicating a position of the at least one marker in the first image. The step of identifying a second ROI comprises calculating a second point indicating a position of the at least one marker in the second image. The step of comparing the first ROI with the second ROI comprises comparing the first point and the second point.

In various embodiments, the step of calculating the first point further comprising:
deriving a first processed image by removing a first set of points of the at least one marker from the first image;
identifying a first region enclosing a second set of points in the first processed image; and
calculating the first point based on the first region; and
the step of calculating the second point further comprising:
   deriving a second processed image by removing a second set of points of the at least one marker from the second image;
   identifying a second region enclosing a second set of points in the second processed image; and
   calculating the second point based on the second region.

The first set of points of the at least one marker are unexpected, useless or redundant information brought by the elbow marker or the shoulder marker. Thus, the first set of points of the at least one marker are abandoned. The second set of the points of the at least one marker are substantially the information of the targeted hand marker. The second set of the points of the at least one marker are kept for subsequent processing.

The first region may be a minimum rectangular border which may enclose the second set of the points of the at least one marker in the first processed image. The second region may be a minimum rectangular border which may enclose the second set of the points of the at least one marker in the second processed image. The minimum rectangle border has two sides in parallel with a horizontal axis and two sides in parallel with a vertical axis. The first region and the second region are determined by the boundary information of the hand marker.

The first region may further be a straight boundary of the hand marker in the first processed image. The second region may further be a straight boundary of the hand marker in the second processed image.

In various embodiments, the first point is a center point or an edge point, and the second point is an edge point. The center point may be a point representing the geometric center of the first region. The edge point of the first ROI may be a point in the first region. The edge point of the second ROI may be a point in the second region. The edge point of the first ROI may further be a point at the straight boundary of the hand marker in the first processed image. The edge point of the second ROI may further be a point at the straight boundary of the hand marker in the second processed image. Therefore, the first point and the second point both are determined based on the boundary information of the hand marker.

In various embodiments, the step of comparing the first ROI (ROI-1) with the second ROI (ROI-2) further comprising determining a distance along a direction between the first point and second point.

The calculated distance refers to position change of the hand marker between the position in the second image and the position in the first image. The calculated distance can reflect the actual displacement of the marker when the patient is doing hand rehabilitation exercise. Since the ROI in the first image and the ROI in the second image are more accurate and reliable, the calculated distance is more accurate and reliable.

In various embodiments, the step of outputting data based on at least the comparing results further comprising determining an angle based on the distance and a distance-angle look-up table.

The distance-angle look-up table may be obtained by doing experiments by moving hand marker to different palmer flexion angle or dorsal flexion angle, each value of the angle is measured and recorded using an angulometer, and each of the corresponding distance between the second image and the first iamge are calculated and recorded based on the first image and second iamge captured at the first position and second position respectively. The distance-angle look-up table is stored in a memory of the processor.

In various embodiments, the step of outputting data based on at least the comparing results further comprising an angle based on the distance, a height of the at least one marker in the first image, and a height-distance-angle look-up table.

The height refers to a height of the area of the marker in the first image along the direction of a vertical axis in the image. Usually, the height of the at least one marker in the first image is more accurate than that in the second image since that the area of the hand marker is in a cubic shape.

In various embodiments, the output data is output to a display. The output data may comprise a flexion/rotation angle being tracked in real time. A motion trajectory may be further obtained based on the flexion/rotation angle in the palmar/volar flexion and dorsal flexion exercise.

When both the target trajectory and the motion trajectory are shown, the user will get information on how well he/she is performing, which will stimulate her in doing her best. This will help in speeding up the rehabilitation process.

### BRIEF DESCRIPTION OF THE DRAWINGS

These and other characteristics, features and advantages of the present invention will become apparent from the following detailed description, taken in conjunction with the accompanying drawings, which illustrate, by way of example, the principles of the invention. The description is given for the sake of example only, without limiting the scope of the invention. The reference umberals given below refer to the attached drawings, in which
Figure 1 shows a prior art rehabilitation device for a rehabilitation exercise;
Figure 2 shows a hand movement exercise being tracked by the rehabilitation device;
Figure 3 shows another hand movement exercise being tracked by the rehabilitation device;
Figure 4 shows a first position of a hand marker being captured by a camera of the rehabilitation device;
Figure 5 shows a second position of a hand marker being captured by the camera of the rehabilitation device;
Figure 6 shows an image captured by the camera at a first position;
Figure 7 shows an image captured by the camera at a second position;
Figure 8 shows an image processing method to obtain a first region and a second region according to an embodiment of the invention;
Figure 9 shows another image processing method to obtain a first region and a second region according to an embodiment of the invention;
Figure 10 shows a method to compare a first ROI and a second ROI according to an embodiment of the invention;
Figure 11 shows another method to compare a first ROI and a second ROI according to an embodiment of the invention;
Figure 12 shows a distance-angle look-up table used for determing the angle according to an embodiment of the invention;
Figure 13 shows a height-distance-angle look-up table used for determing the angle according to an embodiment of the invention;
Figure 14 shows an user interface for outputting the motion angle information according to an embodiment of the invention;
Figure 15 shows a computer for implementing a processor according to an embodiment of the invention;
Figure 16 shows a flowchart of a method tracking rehabiliatation exercise according to an embodiment of the invention;
Figure 17 shows a computer readable medium storing instructions for the computer to perform the method of figure 15 according to an embodiment of the invention; and

The figures are purely diagrammatic and not drawn to scale. In the Figures, elements which correspond to elements already described may have the same reference numerals.

### DETAILED DESCRIPTION OF EMBODIMENTS

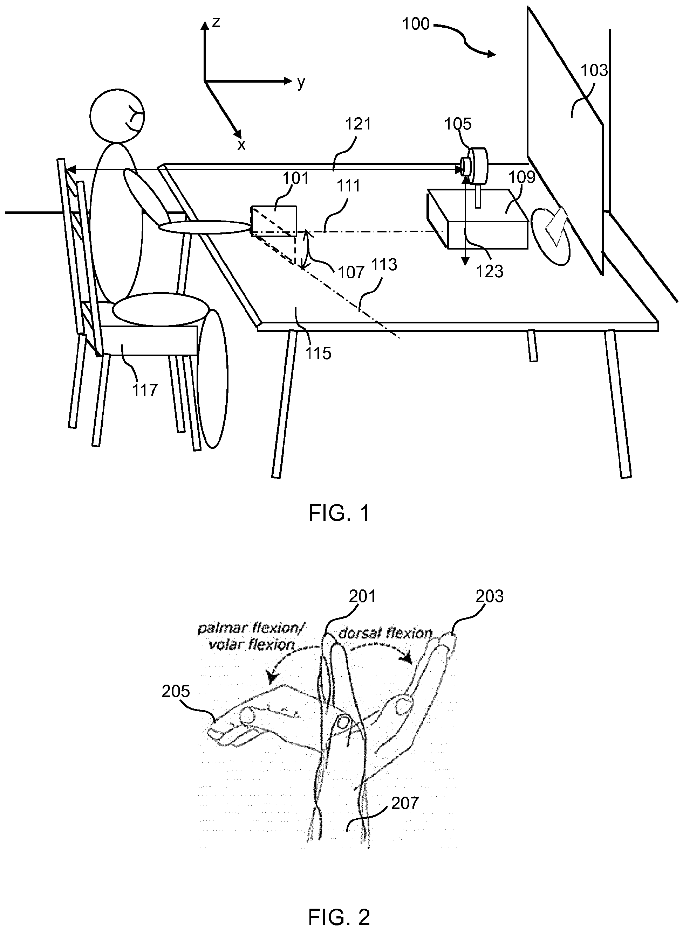

Figure 1 shows a rehabilitation device 100 for a rehabilitation exercise according to an embodiment. The rehabilitation device 100 is placed in a stroke rehabilitation room, e.g., a private rehabilitation hospital, a stroke center of the hospital or a patient's home. When doing rehabilitation exercise, for example, hand exercise, upper limb exercise or lower limb exercise, a subject is standing/sitting in front of the rehabilitation device 100. In this illustrated example, the subject is a post-stroke patient who is doing right hand rehabilitation exercise. In other embodiments, the subject may be a patient with other types of disease that needs to do the rehabilitation exersie or a person in good health who wants to do exercise.

As shown in figure 1, the patient is sitting on a chair 117 and a supporting table 115 is utilized to support his/her hand as well as at least part of the forearm of the patient. The patient can adjust his sitting position so as to find a most comfortable posture. For example, the patient can move his hand forward to gain more support from the supporting table 115. The supporting table 115 is further utilized to support the rehabilitation device 100. Alternatively, two support mechanisms are utilized to support the patient's hand and the rehabilitation device 100 separately.

In some cases, the hand rehabilitation exercise may include a palmar/volar flexion and dorsal flexion movement. More specifically, as shown in figure 2, the patient extends his/her fingers to the maximum extent, and places his/her right hand vertically with respect to the surface of the supporting table 115 with the palm facing left and the dorsa facing right, and vice versa. Starting from the first position 201, the patient bends his/her hand towards left to the position 205 which movement is called palmar/volar flexion. The rotation angle between the hand at the position 205 and the the hand at the position 201 is refered to as a palmar flexion angle. To the opposite, the patient bends his/her hand towards right to the position 203, which movement is called dorsal flextion. The rotation angle between the hand at the position 203 and the hand at the position 201 is refered to as a dorsal flexion angle. The patient's wrist 207 may stand still when doing the palmar/volar flexion and dorsal flexion movement.

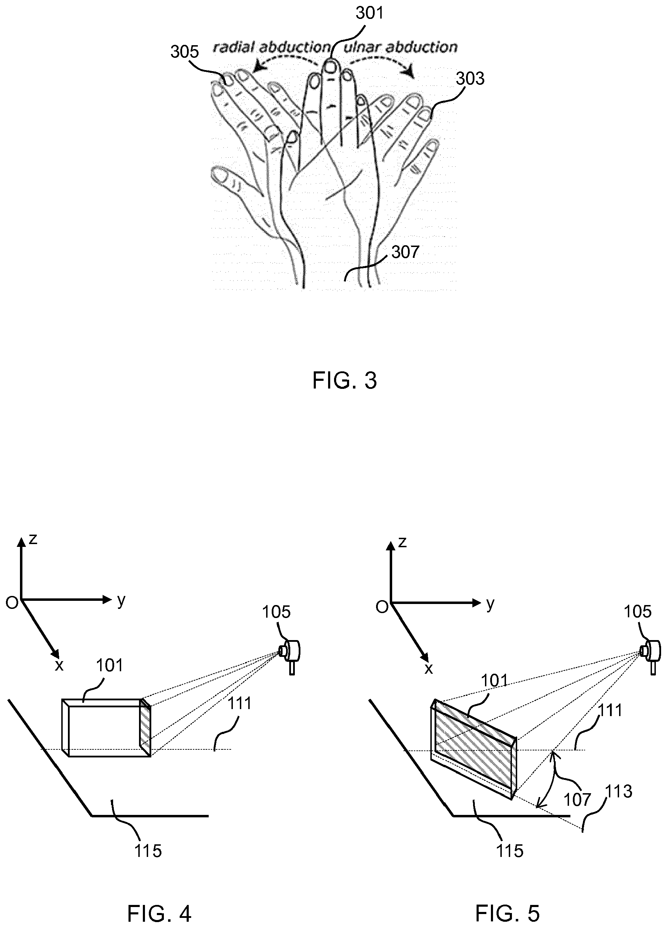

In some cases, the hand rehabilitation exercise may include a radial flexion and ulnar flexion movement. More specifically, as shown in figure 3, the patient extends his/her fingers to the maximum extent and places his/her right hand horizontally with respect to the surface of the the supporting table 115 with the palm downward and the dorsa upward. Starting from the first position 301, the patient swings his/her hand towards left to the position 305, which movement is called radial flexion. The rotation angle between the hand at the position 305 and the the hand at the position 301 is refered to as a radial flexion angle. To the opposite, the patient swings his/her hand towards right to the position 303, which movement is called ulnar flextion. The rotation angle between the hand at the position 303 and the hand at the position 301 is refered to as a ulnar flexion angle. The patient's wrist 307 may stand still when doing the radial flexion and ulnar flexion movement.

In order to have a faster recovery, the patient is required to conduct the repetitive movements and aims at reaching the maximal rotation angle for each movement. How well each movement of the patient is performed needs to be evaluated so as to either encourage the patient to make more effort if the rotation angle does not reach the pre-defined value or to make the patient more confident if the rotation angle exceeds the pre-defined value. Therefore, by accurately determining the rotation angle for each movement and outputting the determined flexion/rotation angle, e.g., displaying the determined rotation angle to the patient, an improved rehabilitation exercise can be achieved as the patient will follow with the determined rotation angle to reach or overreach the repetitie movements. Further explanation regarding how this improvement is achieved by the current invention is described below.

Referring back to figure 1, the palmar/volar flexion and dorsal flexion movement is illustrated. At least one marker 101 is attached to a body part of the patient. In the illustrated example, the at least one marker 101 is a hand marker which is in a cubic shape and is made of retro-flective materials with flexible surface. More specifically, the retro-flective materials can reflect lights from every direction and enable the marker brighter than other parts of the body and background environment. Thus, the movement of the hand marker 101 which defines the movement of the hand can be tracked by a detector such as a camera.

In some cases, a separate splint (not shown in any of figures) is further employed when wearing the hand marker 101. The hand of the patient are fastened onto the splint with fingers being extended as straight as possible. The fastened hand with the splint are inserted into the hand marker 101 from an opening of the hand marker 101. In this way, the hand marker 101 can be attached to the hand smoothly and tightly. In some embodiments, the hand marker 101 may be worn by the user in the same manner as wearing a mitten. In some embodiments, the at least one marker 101 may further include an elbow marker attached to the elbow of the patient and a shoulder marker attached to the shoulder of the patient respectively. The elbow marker and the shoulder marker are in different shapes to fit the elbow's or the shoulder's anatomic structures respectively.

As shown in figure 1, the rehabilitation device 100 includes a processor 109, an image capture unit 105 and a display 103. In the illustrated example, the processor 109, the image capture unit 105 and the display 103 are separate components. For example, the image capture unit 105 may include a camera. The camera 105 is interconnected with the processor 109. The processor 109 is interconnected with the display 103. Alternatively, the camera 105 is in wireless communication with the processor 109 and the processor is in wireless communication with the display 103. In other embodiments, the processor 109, the image capture unit 105 and the display 103 can be assembled as one device and in communication with each other which can be implemented by any means to transmit electronic signals.

The image capture unit 105 comprises a sensor that detects and conveys the information that constitutes an image. The sensor may comprise a CMOS sensor or a CCD sensor. In some embodiments, the image capture unit 105 is an optical instrument for recording or capturing images, which may be stored locally in the processor 109 or transmitted to a remote processor, or both.

The image capture unit 105 may inclue a camera which can capture 2D image of the at least one marker 101. The camera may be a video camera which can capture multiframe 2D images of the at least one marker 101 within a short period of time, for example, in several milliseconds, even when the marker moves very quickly. In some cases, the image capture unit 105 may include two or more cameras, and 3D or 4D image could be reconstructed by processing the images captured by the two or more cameras 105. In some cases, the camera may be a 3D camera to capture the 3D image of the movement of the at least one marker 101.

In one emodiment, a distance 121 between the image capture unit 105 and the chair 117 along y-axis as indicated in figure 1, and a distance 123 between the image capture unit 105 and the surface of the supporting table 115 along z-axis as indicated in figure 1 are fixed respectively. In the illustrated embodiment, the at least one marker 101 can be always kept in the field that can be captured by the image capture unit 105 when the patient is doing hand rehabilitation exercise by adjusting the distance 121 and the distance 123 to two suitable fixed values.

In some cases, the image capture unit 105 may stand still, but the focal length of the image capture unit 105 may be changed by regulating the movement of the camera lens or by using a variable-focal lens.

In some embodiments, the distance 121 and the distance 123 may be adjusted. The image capture unit 105 may be moved forward and backward, up and down, left and right.

It's advantageous that high quality and high resolution images can be captured by adjusting the distance 121 between the target to be captured and the lens of the image capture unit 105 or by adjusting the focus/focal length of the lens.

The processor 109 may be comprised into a thin client terminal such as a pad, a phone, a laptop, or any other types of mobile devices. It is also possible that the processor 109 is comprised into part of a large network system that includes a plurality of computing devices, such as cloud/remote servers. In some embodiments, the image capture unit 105 may be integrated with the processor 109. In other embodiments, the image capture unit 105, the display 103 and the processor 109 may be integrated into a device such as a laptop.

Other peripherals, such as a keyboard, a mouse and/or a USB flash disk (which are not shown in figure 1), may be connected to the processor 109. The connections may be implemented by means of cables or may be by other means, such as wireless communication means, as is known in the prior art.

Figure 4 and figure 5 show a first position and a second position of a hand marker 101 being captured by a camera 105 of the rehabilitation device 100, respectively. Here, a coordinate system of XYZ is used for further illustration as shown in figure 4 and figure 5 respectively. The image capture unit 105 may capture a first image of the at least one marker 101 at a first position along a direction of a dotted line 111 which is in parallel with y-axis as is shown in figure 4. The term "first position" as used herein refers generally to the first position 201 as shown in figrue 2 where the patient starts doing the right hand rehabilitation exercise.

Referring to figure 4, a frontal side of the hand marker 101 is captured in the first image since the frontal side of the hand marker 101 is facing the image capture unit 105. The term "frontal side" as used herein refers generally to the external surface of the hand marker 101 where the finger tips touch/face to internally. In other words, the center line of the frontal side of the marker 101 along z-axis as indicated in figure 4 and the physical center of the image capture unit 105 along z-axis as indicated in figure 4 are in a plane which is in parallel with the ZY plane. In this position, the first position may be easily determined by identifying the center point/center line of the marker along z-axis as indicated in figure 4 in the first image. It's reliable to use this first/initial position as a reference for further use according to an embodiment of the invention.

The image capture unit 105 may capture a second image of the markers at a second position along a direction of a dotted line 113 which is shown in figure 5. The term "second position" as used herein refers generally to the position 203 as showin in figrue 2 which position the patient bends his/her right hand to and at which moment the second image is captured by the image capture unit 105. In the illustrated embodiment, the image capture unit 105 continuously captures images when the patient is doing a real-time exercise. Thus, a subsequent series of second images are captured after the first image is captured at the first position.

More specifically, the surface of the hand maker 101 at the palm side and the frontal side of the hand marker 101 are captured in the second image when the hand marker 101 is moved with the right hand of the patient when doing the dorsal flexion movement. Similarly, the surface of the hand marker 101 at the dorsa side and the frontal side of the hand marker 101 are captured in the second image when the hand marker 101 is moved with the right hand of the patient when doing the palmar/volar flexion movement. A flexion angle 107 between the first position along the direction of the dotted line 111 and the second position along the direction of the dotted line 113 defines the rotation angle in the XY plane and is to be determined as an essential parameter for use in the rehabilitation exercise. A series of values of the flexion angle 107 are determined by analyzing the series of second images and the first image. The determined flexion angle 107 represents the actual hand movement. Thus, a dynamic trajectory of the hand movement can be determined and tracked.

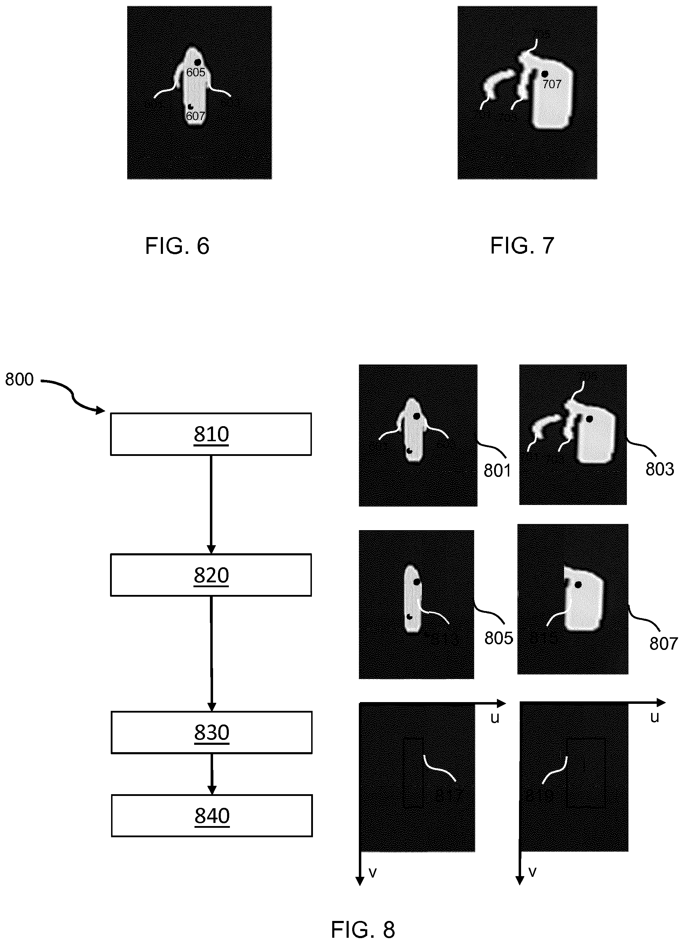

Figure 6 and figure 7 show two images captured by the rehabilitation device 100 at the first position and at the second position separately. It can be seen from the two images that the area of the at least one marker 101 in the first and second images either fails to represent complete information of the hand marker 101 or is mixed with the information of other markers.

More specifically, under some circumstances, it happens that not the whole hand marker is visible due to the marker material and the surface conditions. Hence, the area/sum of marker pixels of the captured image may not correspond to the whole marker. In other words, holes (for instance, elements 605, 607, 707 as shown in figure 6 or figure 7) inside the marker image may diminish the actual area of the hand marker.

In some cases, when the patient is doing upper limb rehabilitation exercese, the hand marker 101, an elbow marker and/or a shouldler marker are worn by the patient. Although the hand marker 101 is the target to be captured by the image capture unit 105, the elbow marker or the shoulder marker may interfere the area of the hand marker 101 in the captured images. As shown in figure 6, the hand marker image is interfered by the elbow marker which is partly overlapped with the hand marker in the first image and is partly visible as the elements 601, 603 shown in the first iamge at the first psition.

In some cases, when doing dorsal flexion with right hand, the hand marker 101 is moved from the first position as shown in figure 4 to the second position as shown in figure 5, the impact of the elbow marker to the hand marker 101 is changed due to the changes of relative relationships of positon and covering between the hand marker 101 and the elbow marker. As shown in figrue 7, part of the elbow marker like elements 701, 703, 705 are visible in the second image at the second position.

Therefore, the area of the markers in the images can't represent the actual information of the hand marker due to the existance of the unexpected, useless or redundant information 601, 603, 701, 703, 705. However, it can be seen that the boundry of the hand marker 101 on the right side is scarcely impacted when the patient bends hand to the right with the right hand. The larger the flexion/rotation angle is, the less interference is induced by the elbow marker since the hand marker 101 covers almost the whole part the elbow marker on the right side from the view of the image capture unit 105 and vice versa.

Therefore, an accurate and reliable method is employed in accordance with an embodiment to be illustrated as below to determine the flexion/rotation angle based on the boundary information instead of using the area information.

Figure 8 shows an image processing method to identify a first region 817 and a second region 819 according to an embodiment of the invention. The first region 817 and the second region 819 are determined for calculating the first ROI and the second ROI espectively. In step 810, the processor 109 receives the first image 801 and the second image 803 from the image capture unit 105.

The first image 801 may be stored in the memory of the processor 109 in the form of binary image. Thus, the binary image comprises a set of points with value 0 and a set of points with value 1. The set of points with the value 0 represent the image of the at least one marker 101 being captured at the first position. The set of points with the value 1 represents the other part or background envirnoments that in the field of the image capture unit 105. In this case, a first set of the points with value 0 in the first image constitute the elements 601 and 603 and a second set of the points with value 0 in the first image constitute the area of the targeted hand marker 101 at the first position. In other words, the first set of the points with value 0 and the second set of the points with value 0 constitute the brighter area in the first image.

Similarly, the second image 803 may be stored in the memory of the processor 109 in the form of binary image. Thus, the binary image comprises a set of points with the value 0 and a set of points with the value 1. The set of points with the value 0 represent the image of the at least one marker 101 being captured at the second position. The set of points with the value 1 represent the other part or background envirnoments that in the field of the image capture unit 105. In this case, a first set of the points with value 0 in the second image 803 constitute the elements 701, 703 and 705 and a second set of the points with value 0 in the second image 803 constitute the area of the targeted hand marker 101 at the second position. In other words, the first set of the points with value 0 and the second set of the points with value 0 constitute the brighter area in the second image.

The processor 109 performs the identification of the first region 817 and the second region 819 with reference to step 820 to step 840 in accordance with this embodiment.

More specifically, in step 820 and step 830, the proessor 109 processes the first image 801 and the second image 803 and derives the first processed image 805 and the second processed image 807 respectively. In an illustrated embodiment, in step 820, the processor 109 derives the first processed image 801 and the second processed image 803 by removing a first set of points 601, 603 of the at least one marker 101 from the first image 801 and removing a second set of points 701, 703, 705 of the at least one marker 101 from the second image 803 respectively. As an example, the processor 109 may process the first image 801 and the second image 803 by using an expansion algorithm after an erosion algorithm respectively. In other embodiments, other algorithms which may achieve the same removal effect can be applied as alternative solutions.

The first processed image 805 includes the second set of points 813 and the second processed image 807 inlcudes the second set of points 815.

Thus, by implementing step 820, the unexpected, useless or redundant information of the elbow marker, e.g., elements 601 and 603, elements 701, 703 and 705 are removed from the first image 801 and the second image 803 respectively. Consequently, the information of the targeted hand marker 101 is kept as shown in the first processed image 803 and the second processed image 805 respectively.

In step 830, the processor 109 performs the detection of the second set of points 813 and the second set of points 815 in the first processed image 805 and the second processed image 807 respectively. More specifically, the second set of points with value 0 in the first processed image 805 is detected and the second set of points with value 0 in the second processed image 807 is detected. Some well known methods in this domain, for example, a connected component labeling method which may detect connected regions is applied to detect the second set of points 813 and 815 respectively. In another example, a blob detection method which may detect the regions that are different in brightness or color is applied to detect the second set of points 813 and 815 respectively. In other embodiments, other detecting method may be applied to detect the second set of points 813 and 815 respectively.

In step 840, the processor 109 performs the identification of the first region 817 which encloses the second set of points 813 in the first processed image 805 and performs the identification of the second region 819 which encloses the second set of points 815 in the second processed image 807 respectively. In one embodiment, the first region 817 may be a minimum rectangular border which encloses the second set of points 813. The second region 819 may be a minimum rectangular border which encloses the second set of points 815. In an embodiment, some well known methods in this domain, for example, an axis-aligned minimum bounding box algorithm is applied to calculate the minimum rectangular border. More specifically, for the given second set of points 813 or 815, the minimum bounding box method subjects to the constraint that the edges of the box are parallel to the coordinate axes (u-axis, v-axis) is utlized as shown in firgure 8.

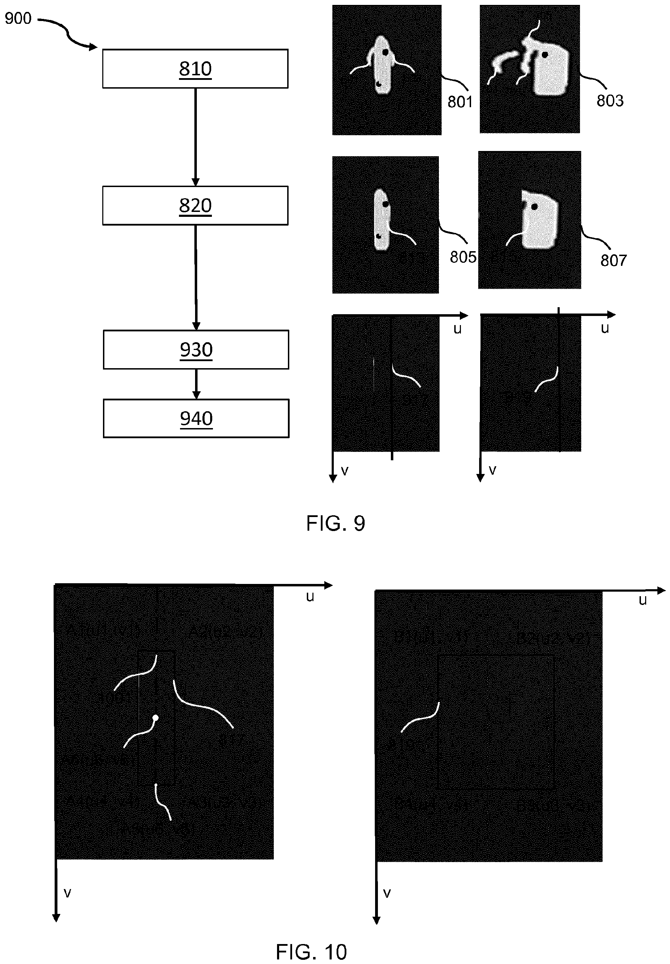

Figure 9 shows another image processing method to identify a first region 917 and a second region 919 according to an embodiment of the invention. The first region 917 and the second region 919 are determined for calculating the first ROI and the second ROI respectively. Steps 810-820 may be the same or similar steps as those shown in figure 8. The detailed description of these steps is omitted herein.

As shown in figure 9, the first region 917 is a straight line of the right boundary of second set of points 813 in the first processed image 805. The second region 919 may further be a straight line of the right boundary of the second set of points 815 in the second processed image 807.

More specifically, in step 930, an edge points detection method is applied to detect boundary of the second set of points 813 and 815 at the right side respectively. As an example, canny edge detector may be used to detect edge points. Thus, edge information of the second set of points 813 and 815 is obtained respectively.

In step 940, a straight line 917 is calculated based on the detected edge points of the second set of points 813 at the right side of the first processed image 805 and a straight line 919 is calculated based on the detected edge points of the set of points 815 at the right side of the second processed image 807. More specifically, position of each detected edge point at u-aixs is used to determine the straight line 917 in parallel with the directin of v-axis. Position of each detected edge point at u-aixs is used to determine the straight line 919 in parallel with the directin of v-axis.

Figure 10 shows a first ROI and a second ROI determined based on the first region 817 and the second region 819 of figure 8 respectively according to an embodiment of the invention. In this embodiment, the first region 817 is a rectangular border which has two sides (sides A1A2 and A3A4) in parallel with a direction of u-axis and two sides (sides A2A3 and A1A4) in parallel with a direction of v-axis. The first region 817 includes four corner points A1(u1, v1), A2(u2, v2), A3(u3, v3) and A4(u4, v4). In this embodiment, the second region 819 is a rectangular border which has two sides in parallel with a direction of u-axis (sides B1B2 and B3B4) and two sides(sides B2B3 and B1B4) in parallel with a direction of v-axis. The second region 819 includes four corner points B1(u1, v1), B2(u2, v2), B3(u3, v3) and B4(u4, v4).

The processor 109 further performs the identification of a first region of interest (ROI-1) of the at least one marker 101 in the first image and the identification of a second region of interest (ROI-2) of the at leat one marker 101 in the second image. In one embodiment, the first ROI comprises a first point which indicates of a position of the at least one marker 101 in the first image 801 and the second ROI comprises a second point which indicates a position of the at least one marker 101 in the second image 803. The processor 109 performs the calculation of the first point and the second point respectively.

In an embodiment, the first point may be a center point and the second point may be an edge point. More specifically, the center point in the first ROI may be a point representing the geometric center of the first region 817, for example, point A6 (u6, v6) which may be determined based on A1(u1, v1), A2(u2, v2), A3(u3, v3) and A4(u4, v4). The center point of the first ROI may further be a point representing a center line (for instance, a dotted line 905) of the first region 817, for example, point A5(u5, v5) which is the middle point of A3(u3, v3) and A4(u4, v4). The edge point of the second ROI may be a point in the second region 819, for example, point B3(u3, v3) or point B2 (u2, v2) at the right side of the second region 819.

In the illustrated embodiment, the processor 109 further performs the comparation of the first ROI and the second ROI. In an embodiment, a first distance (Δd1) along the direction of u-axis when the at least one marker 101 is moved from the first position to the second position is calculated. The first distance (Δd1) defines the displacement between the boundary of the hand marker 101 at the right side in the second position and the center line 905 of the hand marker 101 in the first position along the direction of u-axis. In one example, the comparing result Δd1=|B2_u2-A5_u5|. The term "B2_u2" as used herein refers generally to the position of the point B2 at u-axis. The terms in the same format of "B2_u2" used in the following description have the same explanition. In another example, the comparing result Δd1=|B3_u3-A6_u6|.

In another embodiment, the first point may be an edge point and the second point may be an edge point. More specifically, the edge point of the first ROI may be a point in the first region 817, for example, point A3(u3, v3) or point A2 (u2, v2) at the right side of the first region 817. The edge point of the second ROI may be a point in the second region 819, for example, point B3(u3, v3) or point B2 (u2, v2) at the right side of the second region 819.

In the illustrated embodiment, the processor 109 further performs the comparation of the first ROI and the second ROI. A second distance (Δd2) along the direction of u-axis when the at least one marker 101 is moved from the first position to the second position is calculated. The second distance (Δd2) defines the displacement between the boundary of the hand marker 101 at the right side in the second position and the boundary of the hand marker 101 at the right side in the first position. In one example, the comparing result Δd2=|B3_u3-A3_u3|. In another rexample, the comparing result Δd2=|B2_u2-A2_u2|.

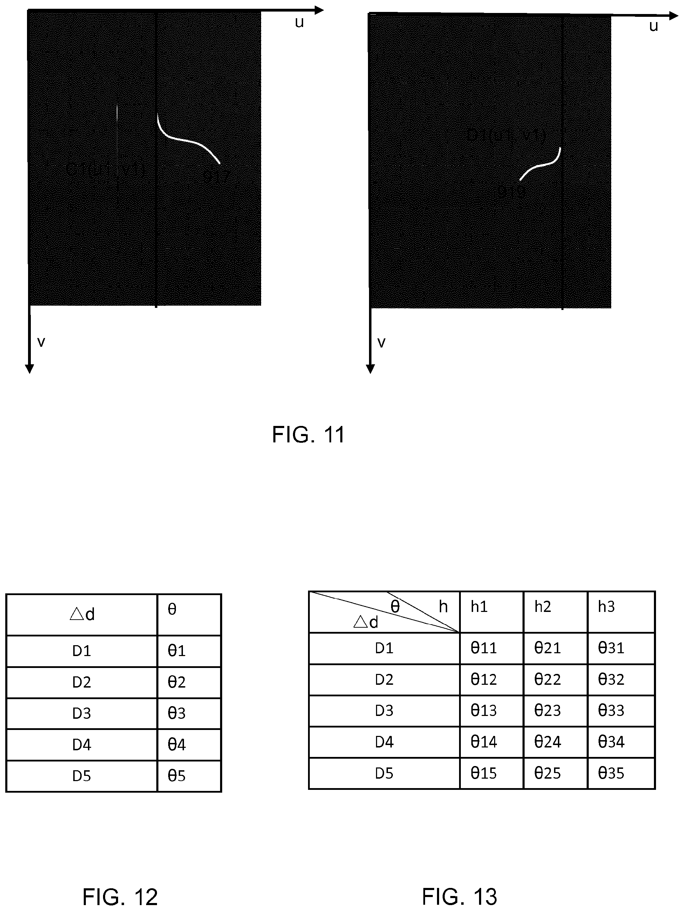

Figure 11 shows a first ROI and a second ROI determined based on the first region 917 and the second region 919 of figure 9 according to an embodiment of the invention. The first region 917 is a straight line along the direction of v-axis. The straight line 917 defines the boundary of the hand marker 101 at the right side at the first position. The second retion 919 is a straight line along the direction of v-axis. The straight line 919 defines the boundary of the hand marker 101 at the right side at the second position.

In one embodiment, the first ROI includes a first point and the second ROI includes a second point. The first point may be an edge point and the second point may be an edge point. More specifically, the edge point of the first ROI may be a point in the first region 917, for example, point C1(u1, v1) in a straight boundary (e.g., the straight line 917). The edge point of the second ROI may be a point in the second region 919, for example, point D1(u1, v1) in a straight boundary (e.g., the straight line 919).

In the illustrated embodiment, the processor 109 performs the comparation of the first ROI and the second ROI. A third distance (Δd3) along the direction of u-axis when the at least one marker 101 is moved from the first position to the second position is calculated. The second distance (Δd3) defines the displacement between the boundary of the hand marker 101 at the right side in the second position and the boundary of the hand marker 101 at the right side in the first position. In one example, the comparing result Δd3=|D1_u1-C1_u1|.

In another embodiemnt, the first ROI comprises a straight boundary, for example, the straight line 917 which is indicative of a position of the at least one marker 101 in the first image 801 and the second ROI comprises the straight line 919 which is indicative of a position of the at least one marker 101 in the second image 803.

In the illustrated embodiment, the processor 109 performs the comparation of the first ROI and the second ROI. In an embodiment, a forth distance (Δd4) along the direction of u-axis when the at least one marker 101 is moved from the first position to the second position is calculated. The second distance (Δd4) defines the displacement between the boundary of the hand marker 101 at the right side at the second position and the boundary of the hand marker 101 at the right side at the first position. In one example, the comparing result Δd4=|line919_u-line917_u|. The term "line919_u" as used herein refers generally to the position of the straght line 919 at u-axis and the term "line917_u" as used herein refers generally to the position of the straght line 917 at u-axis

In other embodiments, the first ROI may further be an area on the boundary of the hand marker in the first processed image 805 as shown in firgure 8. The second ROI may further be an area on the boundary of the hand marker in the second processed iamge 807 as shown in figure 8.

Since it's illustrated as above that the right boundary information is usually accurate without interference of other markers, e.g., the elbow marker and the shoulder marker. It's advantegous of using the information based on the right boundary of the marker in the first/second images when the patient is doing dorsal movement with right hand. Similarly, it's advantegous of using the informatin based on the left boundary of the marker in the first/second iamges when the patient is doing palmar/volar movement with right hand. Similarly, it's advantegous of using the information based on the left boudary of the marker in the first/second images when the patient is doing dorsal movement with left hand. Similarly, it's advantegous of using the informatin based on the right boundary of the marker in the first/second iamges when the patient is doing palmar/volar movement with left hand.

The processor 109 performs the output of data based on at least the comparing results, e.g., the distance Δd1, Δd2, Δd3, and Δd4. More specifically, the output data comprises an angle obtained based on at least the distance Δd.

In some embodiments, after the distance Δd is determined, the processor 109 performs the output of the data based on the comparing results by calculating an angle based on the distance Δd and a distance-angle look-up table. The distance-angle look-up table as shown in figure 12 is stored in the memory of the processor 109 and is obtained by doing experiments.

The distance-angle look-up table may be obtained by doing experiments by moving hand marker to different palmer flexion angle or dorsal flexion angle (θ). For example, starting from the first position, an experimenter moves the hand marker to a series of the second position every 3°, e.g., θ1=3°, θ2=6°, θ3=9°, θ4=12°, θ5=15° and etc. The rehabilitation device 100 may capture the first image at the first position and capture a series of the second images at the series of second position. The angle θ can be measured by using an angulometer and is recorded manually. Each value of distance Δd between the second image and the first image, e.g., D1, D2, D3, D4, D5 and etc. corresponding to each vaule of flexion angle θ is calculated based on the above-mentiond method as illustrated in figure 10 or figure 11. Accordingly, the distance-angle look-up table is obtained.

When the patient starts conducting the palmar/volar flexion and dorsal flexion exercise, the processor 109 performs the calculation of a dynamic distance Δd based on the above-mentioned method as illustrated in figure 10 or figure 11 automatically. The actual flexion angle can be obtained by referring to the distance-angle look-up table based on the calculated distance.

In some embodiments, after the distance Δd is determined, the processor 109 performs the determination of an angle based on the distance, a height of the at least one marker in the first image and a height-distance-angle look-up table. In one embodiment, the height (h) is calculated based on the first region 817 as shown in figure 10 using the corner point's information along the direction of v axis. In an example, h=|A3_v3-A2_v2|. Similarly, the height-distance-angle look-up table as shown in figure 13 is stored in the memory of the processor 109 and is obtained by doing experiments.

More specifically, a person wearing the at least one marker 101 sits in front of the rehbilitation device 100 and conducts the forsal/volar flextion and dorsal flexion movement. The distance along the direction of y-axis as shown in figure 1 between the image capture unit 105 and the frontal side of the hand marker 101 at the first position is adjusted. Thus, different height (h1, h2, h3) in the first images are calculated based on the calculation algorhtim illustrated above. Under each height, distance-angle look-up table is obtained based on the method illustrated in figure 12. Accordingly, the height-distance-angle look-up table is obtained.

When the patient starts conducting the palmar/volar flexion and dorsal flexion exercise, the processor 109 performs the calculation of a dynamic distance Δd based on the above-mentioned method as illustrated in figure 10 or figure 11 automatically. The processor 109 further performs the calculation of a height (h) of the hand marker in the first image. The actual flexion angle can be obtained by referring to the height-distance-angle look-up table based on the calculated distance and the calculated height.

The advantage of adding the height of the at least one marker in the first image as a parameter of the table is that as shown in figure 1, the height of the marker in the first image is different due to the distance along the direction of y-axis as shown in figure 1 between the image capture unit 105 and the hand marker 101 is different even when the patients wear the same hand marker, no mention that different patients may wear different sizes of markers. More specifically, the frontal side of the hand marker forms different sizes of the area in the first iamge because different patients may have different lengths of the forarms or different hemiplegic conditions. When wearing the same hand marker, different distance-angle look-up table is referred to regarding different height of the marker in the first image. The angle can then be obtained in a more accurate and reliable way.

The data may be output to the display 103, a cloud or a controller. In one embodiment, the display 103 is used to show both the target motion trajectory and a motion trajectory of the patient in the rehabilitation exercise. The angle obtained by the method illustrated in this invention represents the motion trajectory of the subject and can be tracked in real time. In one embodiment, the output data is transmitted to the cloud for subsequent processing, for example, outputting feedback on how well the performance of the patient is when doing the hand rehabilitation exercise. In one embodiment, the output data may be transmitted to a controller. The controller may be used to control a mechanism to provide power to help the patient move the hand to the pre-determined extent if the motion angle is less than a value.

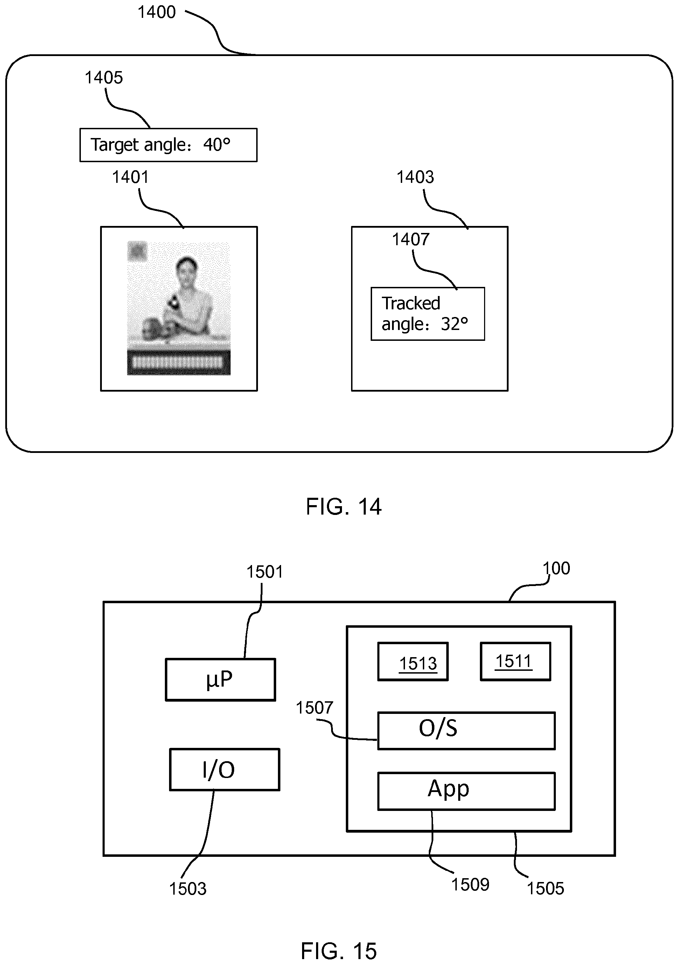

Figure 14 shows an user interface 1400 for outputting the motion angle information. The processor 109 may be arranged to guide the user by displaying, during the rehabilitation exercise, a target motion trajectory 1401 and optionally also a motion trajectory 1403 of the user in the rehabilitation exercise. By showing the target trajectory 1401 and a target angle 1405 on the dispay 103, the user will be guided and stimulated to follow an optimal trajectory. The motion trajectory 1403 of the user can be displayed to give direct visual feedback to the user. The data like a dynamic tracked flexion/rotation angle 1407 is used to form the motion trajectory 1403. In other embodiment, an user interface of an anminated game is designed to show the motion trajectory 1403 using the target angle 1405 as the goal of the game. In order to pass the game, the actual tracked flexion/rotation angle 1407 of the motion trajectory 1403 should exceed the the target angle.

If both the target trajectory and the motion trajectory are shown, the user will get information on how well she is performing, which will stimulate her in doing her best. This will help in speeding up the rehabilitation proces.

Figure 15 illustrates an example of the rehabilitation device 100 as described above. The rehabilitation device 100 includes, but is not limited to, PCs, workstations, laptops, PDAs, palm devices, servers, storages, and the like. Generally, in terms of hardware architecture, the rehabilitation device 100 may include one or more processors 1501, memory 1505, and one or more I/O devices 1503 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1501 is a hardware device for executing software that can be stored in the memory 1505. The processing unit 1501 can be virtually any custom made or commercially available processor, a central processing unit (CPU), a digital signal processor (DSP), or an auxiliary processor among several processors associated with the computer, and the processing unit 1501 may be a semiconductor based microprocessor (in the form of a microchip) or a microprocessor.

The memory 1505 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1505 may incorporate electronic, magnetic, optical, and/or other types of storage media. Note that the memory 1505 can have a distributed architecture, where various components are situated remote from one another, but can be accessed by the processor 1501.

The software in the memory 1505 may include one or more separate programs, each of which comprises an ordered listing of executable instructions for implementing logical functions. The software in the memory 1505 includes a suitable operating system (O/S) 1507, compiler 1511, source code 1513, and one or more applications 1509 in accordance with exemplary embodiments.

The application 1509 comprises numerous functional components such as computational units, logic, functional units, processes, operations, virtual entities, and/or modules. The application 1509 may be a rehabilitation application to communicate with the compiler 1511, source code 1513 and the suitable operating system 1507 to control the processing unit 1501 and I/O devices 1503 to perform the step of monitoring movement of the body parts of the user using the reflected light from the set of markers by analyzing the detected locations in the captured image of the set of markers in the field of view of the image capture unit15. The application 1509 may comprise code means which is adapted, when said program is run on a computer, to perform the steps.

The operating system 1507 controls the execution of computer programs, and provides scheduling, input-output control, file and data management, memory management, and communication control and related services.

Application 1509 may be a source program, executable program (object code), script, or any other entity comprising a set of instructions to be performed. When a source program, then the program is usually translated via a compiler (such as the compiler 1511), assembler, interpreter, or the like, which may or may not be included within the memory 1505, so as to operate properly in connection with the operating system 1507. Furthermore, the application 1509 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, NET, and the like.

The I/O devices 1503 may include input devices such as, for example but not limited to, a mouse, keyboard, scanner, microphone, camera, etc. Furthermore, the I/O devices 1503 may also include output devices, for example but not limited to a printer, display, etc. Finally, the I/O devices 1503 may further include devices that communicate both inputs and outputs, for instance but not limited to, a network interface controller (NIC) or modulator/demodulator (for accessing remote devices, other files, devices, systems, or a network), a radio frequency (RF) or other transceiver, a telephonic interface, a bridge, a router, etc. The I/O devices 1503 also include components for communicating over various networks, such as the Internet or intranet.

When the computer is in operation, the processing unit 1501 is configured to execute software stored within the memory 1505, to communicate data to and from the memory 1505, and to generally control operations of the computer pursuant to the software. The application 1509 and the operating system 1507 are read, in whole or in part, by the processing unit 1501, perhaps buffered within the processing unit 1501, and then executed.

When the application 1509 is implemented in software, it should be noted that the application 1509 can be stored on virtually any computer readable medium for use by or in connection with any computer related system or method.

The system is not limited to hand movements. It may be used for whole body movements, such as bending back and forth or side to side.

The output is described above as a display. Of course, the output may be supplemented with sound, for example including speech generation to provide instructions, performance information or motivational encouragement. Tactile (e.g. vibration) feedback may also be used.

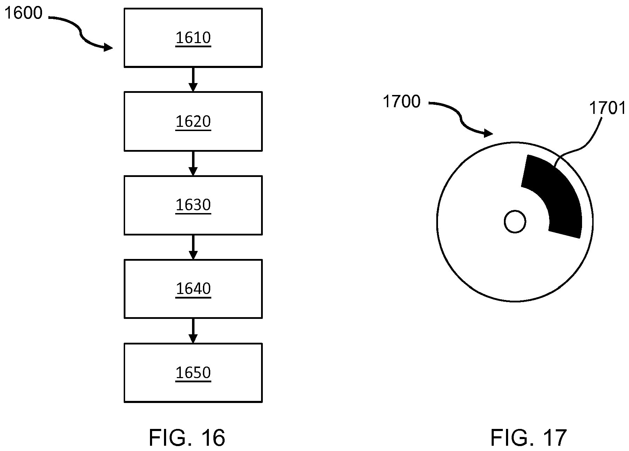

Figure 16 shows a flowchart of a method tracking rehabiliatation exercise. It is noted that the method 1600 may, but does not need to, correspond to an operation of the rehabilitation device 100 as described with reference to figure 1 and others.

The method 1600 comprises in an operation titled "RECEIVING IMAGES", receiving 1610 the first image and the second image from the image capture unit 105. The first image is captured by the image capture unit 105 at the first position and the second image is captured by the image capture unit 105 at the second position.

The method 1600 further comprises, in an operation entitled "IDENTIFYING A FIRST ROI", identifying 1620 a first region of interest (ROI) of the at least one marker in the first image. The method may comprise processing the first image and obtaining the processed image by removing unexpected abundancy information from the first image. The method may further comprise detecting a rectangular border which enclose the marker information of the first processed image. The first ROI comprises a point of one marker obtained based on the rectangular region. The point in the first ROI is a center point or an edge point.

The method 1600 further comprises, in an operation titled "IDENTIFYING A SECOND ROI", identifying 1630 a second region of interest (ROI) of the at leat one marker in the second image. The method may comprise processing the second image and obtaining the processed image by removing unexpected abundancy information from the second image. The method may further comprise detecting a rectangular border which surrounds the marker information of the second processed image. The second ROI comprises a point of one marker obtained based on the rectangular region. The point in the second ROI is an edge point.

The method 1600 further comprises, in an operation titled "COMPARING FIRST ROI AND SECOND ROI", comparing 1640 the first ROI of the first image with the second ROI of the second image. More specifically, the method may comprise comparing the position of the first ROI and the second ROI along the direction of u-axis as shown in figure 10.

The method 1600 further comprises, in an operation titled "OUTPUTTING DATA", outputting 1650 the data based on the comparing results. The method further comparise outputting the data to the display 103 or to a cloud or may be a controller.

It will be appreciated that the above operation may be performed in any suitable order, e.g., consecutively, simultaneously, or a combination thereof, subject to, where applicable, a particular order being necessitated, e.g., by input/output relations.

The method 1600 may be implemented on a computer as a computer implemented method, as dedicated hardware, or as a combination of both. As also illustrated in Figure 17, instructions for the computer, e.g., executable code, may be stored on a computer readable medium 1700, e.g., in the form of a series 1701 of machine readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. figure 17 shows an optical disc 1700.

It will be appreciated that the above description for clarity has described embodiments of the invention with reference to different functional units and processors. However, it will be apparent that any suitable distribution of functionality between different functional units or processors may be used without deviating from the invention. For example, functionality illustrated to be performed by separate units, processors or controllers may be performed by the same processor or controllers. Hence, references to specific functional units are only to be seen as references to suitable means for providing the described functionality rather than indicative of a strict logical or physical structure or organization. The invention can be implemented in any suitable form including hardware, software, firmware or any combination of these.

It is noted, that in this document the word 'comprising' does not exclude the presence of other elements or steps than those listed and the word 'a' or 'an' preceding an element does not exclude the presence of a plurality of such elements, that any reference signs do not limit the scope of the claims, that the invention may be implemented by means of both hardware and software, and that several 'means' or 'units' may be represented by the same item of hardware or software, and a processor may fulfill the function of one or more units, possibly in cooperation with hardware elements. Further, the invention is not limited to the embodiments, and the invention lies in each and every novel feature or combination of features described above or recited in mutually different dependent claims.

## Claims

1. A rehabilitation device (100) for a rehabilitation exercise, the device comprising:
at least one marker (101) configured to be attached to at least one body part of a subject;
an image capture unit (105) configured to capture a first image (801) of the at least one marker (101) at a first position and a second image (803) of the at least one marker (101) at a second position; and
a processor (109) configured to:
receive the first image and the second image from the image capture unit (105);
identify a first region of interest (ROI-1) of the at least one marker (101) in the first image;
identify a second region of interest (ROI-2) of the the at least one marker (101) in the second image;
compare the first ROI (ROI-1) with the second ROI (ROI-2); and
output data based on at least the comparing results.

2. The rehabilitation device of claim 1, wherein the processor (109) is further configured to:
identify the first ROI (ROI-1) by calculating a first point indicating a position of the at least one marker (101) in the first image;
identify the second ROI (ROI-2) by calculating a second point indicating a position of the at least one marker (101) in the second image; and
compare the first point with the second point.

3. The rehabilitation device of claim 2, wherein the first point is a center point or an edge point, and wherein the second point is an edge point.

4. The rehabilitation device of claim 2, wherein the processor (109) is further configured to compare the first ROI (ROI-1) with the second ROI (ROI-2) by calculating a distance along a direction between the first point and the second point.

5. The rehabilitation device of claim 4, wherein the processor (109) is further configured to output the data based on the comparing results by at least determining an angle based on the distance and a distance-angle look-up table.

6. The rehabilitation device of claim 4, whrerin the processor (109) is further configured to output the data based on the comparing results by at least determining an angle based on the distance, a height of the at least one marker in the first image, and a height-distance-angle look-up table.

7. The rehabilitation device of claim 5 or 6, wherein the processor (109) is further configured to output the angle.

8. The rehabilitation device of claim 1, wherein the processor (109) is further configured to output the data to a display.

9. A computer-implemented method (1600) for rehabiliatation exercise, comprising:
receiving (1610) a first image and a second image;
identifying (1620) a first region of interest (ROI) (ROI-1) of at least one marker in the first image;
identifying (1630) a second region of interest (ROI) (ROI-2) of the at leat one marker in the second image;
comparing (1640) the first ROI (ROI-1) with the second ROI (ROI-2); and
outputting (1650) data based on at least the comparing results.

10. The method of claim 9, wherein:
the step of identifying (1620) the first ROI (ROI-1) comprises calculating a first point indicating a position of the at least one marker (101) in the first image;
the step of identifying (1630) a second ROI (ROI-2) comprises calculating a second point indicating a position of the at least one marker (101) in the second image; and
the step of comparing (1640) the first ROI (ROI-1) with the second ROI (ROI-2) comprises comparing the first point and the second point.

11. The method of claim 10, wherein:
the step of calculating the first point further comprising:
deriving a first processed image by removing a first set of points (601, 603) of the at least one marker (101) from the first image;
identifying a first region (817) enclosing a second set of points (813) in the first processed image (805); and
calculating the first point based on the first region (817); and
wherein the step of calculating the second point further comprising:
deriving a second processed image by removing a second set of points (701, 703,705) of the at least one marker (101) from the first image;
identifying a first region (817) enclosing a second set of points (815) in the second processed image (807); and
calculating the second point based on the second region (819).

12. The method of claim 10, wherein the step of comparing the first ROI (ROI-1) with the second ROI (ROI-2) further comprising determining a distance along a direction between the first point and second point.

13. The method of claim 11, wherein the step of outputting data based on at least the comparing results further comprising determining an angle based on the distance and a distance-angle look-up table.

14. The method of claim 11, wherein the step of outputting data based on at least the comparing results further comprising an angle based on the distance, a height of the at least one marker in the first image, and a height-distance-angle look up table.

15. Computer program product for operating a rehabilitation device, which program is operative to cause a processor to perform the method as claimed in claims 9-14.
